# EUROPÄISCHE PATENTSCHRIFT

(11) **EP 0 671 977 B1**
(45) Veröffentlichungstag und Bekanntmachung des Hinweises auf die Patenterteilung: **09.10.1996**
(21) Anmeldenummer: 94900719.9
(22) Anmeldetag: 24.11.1993
(51) Int. Cl.: B01J 29/40, B01J 29/89, B01J 37/02

(54) **OXIDATIONSKATALYSATOREN**
OXYDATION CATALYSTS
CATALYSEURS D'OXYDATION

(30) Priorität: 03.12.1992 DE 4240692; 03.12.1992 DE 4240693; 03.12.1992 DE 4240691; 03.12.1992 DE 4240698
(43) Veröffentlichungstag der Anmeldung: 20.09.1995
(73) Patentinhaber: KataLeuna GmbH Catalysts, 06236 Leuna (DE)
(72) Erfinder: SCHÖDEL, Rainer, D-06122 Halle (DE); BIRKE, Peter, D-06124 Halle (DE); GEYER, Reinhard, D-06126 Halle (DE); KRAAK, Peter, D-04159 Leipzig (DE); MÜLLER, Willibald, D-06217 Merseburg (DE); NEUBAUER, Hans-Dieter, D-06217 Merseburg (DE); PESTER, Rolf, D-06231 Bad Dürrenberg (DE); VOGT, Fritz, D-06124 Halle (DE); WENDLANDT, Klaus-Peter, D-06217 Merseburg (DE)
(74) Vertreter: Schinke, Herbert, Dr. Dr., Patentanwaltskanzlei
(86) Internationale Anmeldenummer: DE9301117
(87) Internationale Veröffentlichungsnummer: WO9412277

(56) Entgegenhaltungen:
- EP-A- 0 102 097
- EP-A- 0 267 362
- EP-A- 0 299 430
- EP-A- 0 314 582
- EP-A- 0 469 662
- EP-A- 0 511 739
- US-A- 4 800 187

## Beschreibung

### Technisches Gebiet

Die Erfindung betrifft neue Oxidationskatalysatoren, Verfahren zu deren Herstellung sowie die Verwendung dieser Katalysatoren für Oxidationsreaktionen unter milden Bedingungen, wie Temperaturen von 20 bis 120 °C und Drücken von gleich oder höher als atmosphärischer Druck.

### Stand der Technik

Es sind bereits eine Vielzahl von Oxidationskatalysatoren bekannt, die für Oxidationsreaktionen mit H₂O₂ als Oxidationsmittel eingesetzt werden. So ist in der DE-OS 3 309 669 ein Katalysator aus zeolithischen Materialien mit Fremdelementen beschrieben. Als modifizierende Fremdelemente werden Cr, Be, Ti, V, Mn, Fe, Ca, Zn, Rh, Ag, Sn, Sb und B angegeben.

In den letzten Jahren wurden als Qxidationskatalysatoren bevorzugt kristalline Titansilikalite eingesetzt. So sind folgende Anwendungsfälle bekannt: die Synthese von Glykolmonomethyläther (EP 100 118), die Epoxidierung von Monoolefinen (EP 100 119), die Epoxidierung von Diolefinen zu Monoepoxiden (EP 190 609), die Umwandlung von Styrol zu β-Phenylaldehyd (EP 102 097), die Hydroxylierung von Aromaten (GB 2 116 974), die Oxidation von Alkanen in Alkohole und Ketone (Nature, 345 (1990) 240), die Oxidation von Alkoholen zu Aldehyden bzw. Ketonen (EP 102 655) sowie die Umwandlung von Cyclohexanon mit NH₃ und H₂O₂ zum Oxim (EP 208 311 und 226 257).

Zur Herstellung der kristallinen Titansilikalite werden in der Literatur verschiedene Verfahren angeführt. Im US 4 410 501 werden zwei Herstellungsverfahren beschrieben. Beide Verfahren beinhalten das Herstellen eines TiO₂-SiO₂-Gels, das in Gegenwart von Tetrapropylammoniumhydroxid (TPAOH) und Wasser unter hydrothermalen Bedingungen zum kristallinen Titansilikalit (TS-1) umgewandelt wird. Als Ausgangsprodukte für die Gelbildung dienten Tetraethylorthosilikat (TEOS) und Tetraethylorthotitanat (TEOT) bzw. kolliodales SiO₂ und Tetrapropylammoniumperoxotitanat. Der Einsatz von Tetrabutylorthotitanat als TiO₂-Quelle wird in J. Catal. 130, (1991), 1, beschrieben.

Weiterhin können Titansilikalite durch Hochtemperaturbehandlung von H-ZSM 5 oder Silikalit-1 mit TiCl₄ hergestellt werden (Catal. Lett. 13 (1992) 229). Das EP 299 430 schützt ein Herstellungsverfahren von Titansilikalit, wonach amorphes SiO₂ mit einer Titanverbindung imprägniert und anschließend in Gegenwart eines Templates zum Titansilikalit kristallisiert wird. Die dabei erhaltenen Titansilikalite besitzen eine geringe Selektivität in der Bildung von Cyclohexanonoxim. Erst durch eine aufwendige Aktivierung des Katalysators mit H₂O₂ und H₂SO₄ wird die Selektivität der Oximbildung verbessert; die erreichten Selektivitäten sind jedoch für eine technische Nutzung zu gering.

Im EP 314 582 wird eine ökologisch ungünstige Variante der Titansilikalitsynthese mit den Reagenzien SiO₂, Oxalsäure, Titanoxalat, Tetrapropylammoniumbromid, Ammoniumfluorid und Zeolithkristallisationskeimen beschrieben, die allerdings zu großen Zeolithkristallen führt. Die hierbei erhaltenen Zeolithkristallitgrößen von 15 bis 20 µm sind zwar günstig für das Sedimentationsverhalten, zeigen jedoch in zahlreichen Oxidationsreaktionen eine zu geringe Aktivität.

Die hydrothermale Umwandlung von gemeinsam gefällten TiO₂-SiO₂-Produkten in Gegenwart von Templaten zu Titansilikalit wird im EP 311 983 geschützt.

Alle diese aus dem Stand der Technik bekannten Titansilikalitkatalysatoren besitzen den Nachteil, daß sie kostenintensiv sind, eine ungünstige Kombination zwischen leicht zugänglicher Zeolithoberfläche und Katalysatorpartikelgröße aufweisen und damit schlecht handhabbar sind. Außerdem ist zum Einstellen hoher Aktivitäts- und Selektivitätswerte eine Aktivierung mit H₂O₂ und H₂SO₄ vor dem Einsatz erforderlich.

### Darstellung der Erfindung

Die Aufgabe der vorliegenden Erfindung bestand somit darin, kostengünstigere Oxidationskatalysatoren zu entwickeln, die zudem noch ein verbessertes Aktivitäts- und Selektivitätsverhalten aufweisen.

Diese Aufgabe wird durch ein neues katalytisches System, bestehend aus auf Aktivkohle oder auf Metalloxid in situ kristallisiertem, geträgertem Titansilikalit mit MFI-Struktur gelöst.

Der Titansilikalitgehalt der erfindungsgemäßen Katalysatoren liegt in den Grenzen von 1 bis 90 Masse-%; zweckmäßigerweise beträgt er 10 bis 90 Masse-%. Für auf Aktivkohle geträgerte Titansilikalite liegt der bevorzugte Titansilikalitgehalt im Bereich von 40 bis 60 Masse-%, für auf Metalloxid geträgerte Titansilikalite beträgt der bevorzugte Titansilikalitgehalt 30 bis 50 Masse-%.

Das Si-Ti-Atomverhältnis in der geträgerten Phase beträgt 10 bis 100.

Als Oxide, auf die die Titansilikalite aufkristallisiert werden, werden Al₂O₃, SiO₂, TiO₂, ZrO₂ oder Al₂O₃ . SiO₂ eingesetzt. Es können aber auch beliebige Gemische der aufgeführten Oxide verwendet werden.

Es wurde überraschend gefunden, daß Titansilikalite, auf Aktivkohle oder oxidische Träger aufgebracht, und zwar auf diese Träger in situ aufkristallisiert (im weiteren als "geträgert" bezeichnet), eine wesentlich verbesserte Aktivität und Selektivität aufweisen gegenüber den ungeträgerten Titansilikaliten. Darüber hinaus besitzen sie auch noch gute Sedimentationseigenschaften, da die Katalysatorkorngröße bei < 63 µm liegt, mit einem Maximum in der Korngrößenverteilung im Bereich von 8 bis 30 µm. Die Korngröße der reinen Titansilikalite liegt dagegen bei ≦ 5 µm.

Die Ursache für diese überraschende Wirkung wird in der vorhandenen Wechselwirkung zwischen dem Träger und dem Titansilikalit gesehen. Gegenüber den reinen, trägerfreien Titansilikaliten treten stark abweichende physikalischchemische Merkmale auf. Diese sind: ein signifikant kleineres Elementarzellenvolumen in der geträgerten Titansilikalitphase sowie eine Verschiebung der Si-O-Ti-Bande bei 960 cm⁻¹ zu kleineren Wellenzahlen. Signifikant kleiner bedeutet, daß die Verringerung außerhalb der Fehlergrenze der Messung für die reinen Titansilikalite liegt.

Nach Literaturangaben (J. of Catalysis 130, (1991), 1) nimmt das Elementarzellenvolumen des Titansilikalits bei einem Ti/(Ti+Si)-Atomverhältnis von 0 einen Wert von 5,3447 nm³ an und steigt im bereich der Ti(Ti+Si)-Atomverhältnisse von 0 bis 0,091 linear mit zunehmendem Ti-Gehalt an. Bei einem Ti(Ti+Si)-Verhältnis von 0,091 beträgt der Wert des Elementarzellenvolumens 5,3965 nm³. Sowohl an den auf Aktivkohle als auch an den auf Metalloxid-Trägern fixierten Titansilikaliten wurde eine signifikante Schrumpfung der Elementarzelle nachgewiesen (vgl. Tabelle 1), die auf eine strukturbeeinflussende Wirkung des Trägers auf die zeolithische Komponente zurückgeführt wird.

In der Tabelle 1 sind auch die Ergebnisse der IR-spektroskopischen Messungen von auf Aktivkohle geträgerten Titansilikaliten enthalten. Bei den in den nachfolgenden Beispielen beschriebenen erfindungsgemäßen Katalysatoren A, B und C liegt die Bande der Si-O-Ti-Schwingung zwischen 946 und 949 cm⁻¹ und beim reinen Titansilikalit V sowie beim vorsichtig abgebrannten Trägerkatalysator A 1 bei 967 bzw. 966 cm⁻¹.

Die Herstellung der erfindungsgemäßen Titansilikalit-Aktivkohle-Trägerkatalysatoren kann nach verschiedenen Verfahren erfolgen. So ist es beispielsweise möglich, auf die Aktivkohle ein TiO₂-SiO₂-Gemisch durch gemeinsames Auffällen aufzubringen und dann eine hydrothermale Behandlung in Gegenwart von Tetrapropylammoniumhydroxid über einen Zeitraum von 24 bis 240 Stunden bei Temperaturen von 150 bis 200 °C anzuschließen.

Eine weitere Herstellungsvariante besteht in dem Auffällen von SiO₂ und Aktivkohle und nachfolgendem Tränken des C-SiO₂-Gemisches mit einer Titanverbindung und anschliessende hydrothermale Behandlung, wie oben angegeben.

Als SiO₂-Quelle dienen z. B. Wasserglas und Si(OC₂H₅)₄. Geeignete Titanverbindungen sind bekanntermaßen TiOCl₂ und Ti(OC₂H₅)₄.

Die Herstellung der Titansilikalit-Metalloxid-Trägerkatalysatoren kann durch das gemeinsame Auffällen eines TiO₂-SiO₂-Gemisches in Gegenwart von Tetrapropylammoniumhydroxid über einen Zeitraum von 48 h bis 240 h bei Temperaturen von 150 °C bis 200 °C erfolgen.

Eine weitere Herstellungsvariante ist das Auffällen von SiO₂ auf den Träger mit anschließendem Tränken des Träger-SiO₂-Gemisches mit einer Titanverbindung und nachfolgender hydrothermaler Behandlung in Gegenwart eines Templates.

Eine dritte Möglichkeit ist das Auftränken eines titanhaltigen Kieselsols auf das Metalloxid und nachfolgende hydrothermale Behandlung.

Als TiO₂-Quelle können ebenfalls TiOCl₂ und Ti(OC₂H₅)₄ und als SiO₂-Quelle Wasserglas und Si(OC₂H₅)₄ dienen.

Gegenstand der Erfindung ist weiterhin die Verwendung der erfindungsgemäßen Katalysatoren für Oxidationsreaktionen unter milden Bedingungen, wie Temperaturen von 20 bis 120 °C und Drücken gleich oder höher als Atmosphärendruck und unter Einsatz von H₂O₂.

Weitere Einsatzmöglichkeiten für die erfindungsgemäßen Katalysatoren sind auch in den folgenden Reaktionen mit verbesserter Effektivität gegeben:
- Hydroxylierung von Aromaten
- Oxidation von gesättigten Kohlenwasserstoffen
- Oxidation von Olefinen
- Oxidation von Allylalkohol
- Oxidation von Alkoholen
Besonders bevorzugt werden diese Katalysatoren für die Herstellung von Oximen durch katalytische Umwandlung der entsprechenden Carbonylverbindung eingesetzt.

Es wurde nämlich überraschenderweise gefunden, daß diese Trägerkatalysatoren bei der Umwandlung von Ketonen mit NH₃ und H₂O₂, auch ohne vorherige Aktivierung mit H₂O₂ und H₂SO₄, eine deutlich höhere Aktivität aufweisen als reine Titansilikalitkatalysatoren (vgl. Tabelle 2).

Die Durchführung des Verfahrens zur Herstellung von Oximen durch Umwandlung der entsprechenden Carbonylverbindung mit Ammoniak und H₂O₂ erfolgt bei Einsatz des auf Aktivkohle geträgerten erfindungsgemäßen Katalysators bei H₂O₂ : Carbonylverbindung-Molverhältnissen von 0,8 bis 1,2, NH₃ : Carbonylverbindung-Molverhältnissen von 1,2 bis 2,5, Temperaturen von 20 bis 120 °C, einem Druck gleich oder höher als atmosphärischer Druck in Wasser und einem organischen Lösungsmittel unter intensivem Rühren.

Wird ein auf Metalloxid geträgerter erfindungsgemäßer Katalysator dafür verwendet, dann werden H₂O₂ : Carbonylverbindung-Molverhältnisse von 0,8 bis 2,0 eingestellt.

Bei Durchführung dieses Verfahrens werden der Katalysator, das Lösungsmittel und Ammoniak vorgelegt und H₂O₂ sowie die Carbonylverbindung getrennt über Dosiervorrichtungen gleichzeitig zugeleitet, wobei die Dosiergeschwindigkeiten für H₂O₂ und Carbonylverbindung 0,5 Mol bzw. 0,4 Mol pro kg Katalysator pro Stunde nicht überschreiten sollten. Hohe Oximausbeuten werden erhalten, wenn die Katalysatorkonzentration für die auf Aktivkohle geträgerten Titansilikalite im Bereich von 0,02 bis 30 g pro Mol Carbonylverbindung liegt. Als besonders günstig haben sich dafür Katalysatorkonzentrationen von 1 bis 6 g Katalysator je Mol Carbonylverbindung erwiesen. Für die auf Metalloxid geträgerten Titansilikalite sollten die Katalysatorkonzentrationen im Bereich von 0,05 bis 30 g, vorzugsweise 1 bis 8 g, pro Mol Carbonylverbindung liegen.

Zum Erreichen hoher Selektivitäten und Aktivitäten sind ferner Reaktionstemperaturen von 60 bis 90 °C und geringe Überdrücke von 27 bis 93 kPa (200 bis 700 Torr) günstig.

Diese hohe Ammoximations- bzw. Oxidationsaktivität wird - wie schon vorn ausgeführt - auf eine durch Wechselwirkung des Titansilikalits mit dem Träger verbundene Verzerrung des Zeolithgitters zurückgeführt.

Bei Verwendung der erfindungsgemäßen Katalysatoren für die obengenannten Reaktionen ist es wesentlich, daß der Titansilikalitgehalt im Trägerkatalysator 1 bis 90 Masse-%, zweckmäßigerweise 10 bis 90 Masse-% und vorzugsweise 40 bis 60 Masse-% bei Einsatz von Aktivkohle als Trägermaterial und 30 bis 50 Masse-% bei Einsatz von Metalloxid als Trägermaterial beträgt, und das Si-Ti-Atomverhältnis im Bereich von 10 bis 100 liegt.

Die Größe der Katalysatorteilchen liegt unterhalb 63 µm. Als besonders günstig hat sich für die Durchführung der genannten Verfahren unter technischen Bedingungen ein Katalysatorkornspektrum im Bereich von 8 bis 30 µm erwiesen.

Die Erfindung wird anhand der Ausführungsbeispiele näher erläutert.

Die nach den nachfolgenden Beispielen 1 bis 4 hergestellten Katalysatoren wurden mit den Methoden a, b und c, die nach den Beispielen 5 bis 9 hergestellten Katalysatoren mit den Methoden b und c charakterisiert:
a) IR-Spektroskopie zur Bestimmung der Lage der Si-O-Ti-Schwingungsbande.
   (KBr - Preßtechnik)
b) Röntgenographische Ermittlung des Elementarzellenvolumens (EZV) des geträgerten Aktivkohle-Titansilikalits.
   Die Berechnung des Elementarzellenvolumens der zeolithischen Komponente erfolgt aus der röntgenographischen Präzisionsvermessung der fünf intensitätsstarken Interferenzen (501), (051), (151), (303) und (133) im Winkelbereich 2ϑ = 23,0 ° bis 2ϑ = 25,8 ° mit Ni-gefilterter Cu-Kα-Strahlung an einem Horizontalzählrohrgoniometer HZG 4/B der Freiberger Präzisionsmechanik GmbH (Aufnahmebedingungen: Schrittweite Δ2ϑ = 1/100 °, Zählzeit/Meßpunkt t=60 sec., Divergenzblende b_{div.} = 1.09 mm; Zählrohrblende b_{z}=0,13 mm). Zur Einstellung eines definierten Wassergehaltes wird die Probe vor der Messung mindestens 12 h über einer gesättigten MgCl₂-Lösung gelagert. Bestimmt werden die durch einen inneren Standard (Korund) verabsolutierten Linienlagen mittels eines Peak-Suchprogrammes. Das für die Berechnung des Elementarzellenvolumens eingesetzte Rechenprogramm geht von einer monoklinen Gittersymmetrie aus, in die sich das orthorhombische System des Titansilikalits mit MFI-Struktur als Spezialfall einfügt. (MFI: IUPAC-Name für calcinierte ZSM 5-Zeolithe)
c) Bestimmung der katalytischen Aktivität in der Umwandlung von Cyclohexanon mit Ammoniak und H₂O₂ zum Cyclohexanonoxim.

In der Tabelle 1 sind die Elementarzellenvolumina für einen reinen Titansilikalit als Vergleichskatalysator V sowie für die erfindungsgemäßen Titansilikalit-Aktivkohle-Trägerkatalysatoren A bis C, außerdem für einen trägerfreien Titansilikalit, der durch 20-stündiges Abbrennen des Kohlenstoffes aus dem erfindungsgemäßen Katalysator A bei 500 bis 550 °C erhalten wurde und für die auf Metalloxid geträgerten Katalysatoren D bis J angegeben. Bei den Synthesen der in der Tabelle 1 angeführten erfindungsgemäßen Katalysatoren wurde ein Ti/(Ti+Si)-Wert von 0,06 eingestellt.

Die Daten in der Tabelle zeigen, daß die Elementarzelle der geträgerten Titansilikalite einer starken Schrumpfung unterliegt. Nach vorsichtigem Abbrennen der Aktivkohle liegt das Elementarzellenvolumen wieder in der gleichen Größe wie beim reinen Titansilikalit.

Die strukturbeeinflussende Wirkung des Trägers auf die zeolithische Komponente zeigen auch die Ergebnisse der IR-spektroskopischen Messungen. Bei den erfindungsgemäßen Katalysatoren A, B und C liegt die Bande der Si-O-Ti-Schwingung bei 947 bzw. 946 bzw. 949 cm⁻¹ und beim reinen Titansilikalit (Katalysator V) sowie beim vorsichtig abgebrannten Trägerkatalysator A 1 bei 967 bzw. 966 cm⁻¹.

In der Tabelle 2 sind die katalytischen Aktivitäten und Selektivitäten der erfindungsgemäßen Katalysatoren zusammengefaßt. Die Ergebnisse veranschaulichen deren Vorzüge, insbesondere hinsichtlich der hohen Oximausbeute bei hoher Selektivität.

Die weiteren Vorteile bestehen darin, daß
- diese Katalysatoren keine vorherige Aktivierung mit H₂O₂ und H₂SO₄ benötigen,
- der Katalysatorverbrauch niedriger liegt,
- weniger Nebenprodukte anfallen und
- ein geringerer Gehalt an kostenintensiven Titansilikaliten erforderlich ist.

Zudem zeigte sich bei der Anwendung, daß die geträgerten Titansilikalite besser handhabbar sind, bedingt durch die Größe der Teilchen von ≦ 63 µm, mit einem Maximum der Teilchengrößenverteilung im Bereich von 8 bis 30 µm.

### Beispiel 1 (Vergleichsbeispiel)

Zu 54,4 g Tetraethylorthosilikat wurden unter Stickstoffspülung und Rühren 2,4 g Tetraisopropyltitanat gegeben. Dieses Gemisch wurde danach tropfenweise mit 120 g Tetrapropylammoniumhydroxidlösung (20 %) versetzt. Das Gemisch wurde eine Stunde bei Raumtemperatur belassen und anschließend langsam auf 78 °C erhitzt, eine Stunde bei dieser Temperatur gehalten und nachfolgend zur Entfernung des Isopropanols auf 98 °C erhitzt. Nach dem Abkühlen wurde das Flüssigkeitsvolumen mit destilliertem Wasser auf 200 ml gebracht. Das erhaltene Produkt wurde in einem Autoklaven bei 175 °C und unter autogenem Druck über eine Zeitdauer von 10 Tagen behandelt. Anschließend wurde das Reaktionsprodukt auf Raumtemperatur abgekühlt, abfiltiert, bis zum pH=7 gewaschen, 15 h bei 120 °C getrocknet und danach 10 h bei 420 °C calciniert. Danach wurde der Katalysator bei 70 °C zwei Stunden mit einem Gemisch aus 10 cm³ H₂O₂ (30 Masse-%) und 100 ml H₂SO₄ (5 Masse-%) unter Rühren behandelt.
Anschließend wurde die Flüssigkeit durch Dekantieren abgetrennt und die H₂O₂-H₂SO₄-Behandlung noch zweimal wiederholt. Das kristalline Produkt wurde nachfolgend bis zum pH 7 gewaschen, 15 h bei 120 °C getrocknet und dann zwei Stunden bei 550 °C getempert.
Das hierbei erhaltene Produkt wird in der Tabelle 2 als Katalysator V bezeichnet (Teilchengröße ≦ 5 µm).

### Beispiel 2

Zu 78,66 g Tetraethylorthosilicat werden unter Inertgasspülung und Rühren 2,21 g Tetraethylorthotitanat gegeben. Anschließend setzt man diesem Gemisch, ebenfalls unter lnertgasspülung, 172,5 g 20%-ige Tetrapropylammoniumhydroxidlösung zu. Dieses Gemisch wird eine Stunde unter Rühren bei 78 °C behandelt und mit 157,32 g Wasser verdünnt. In diese Lösung wurden 15 g säuregewaschene Fichtenholz-Aktivkohle (DARCO, Teilchengröße < 32 µm) eingetragen. Die homogene Suspension wird bei Raumtemperatur in einen teflonausgekleideten Autoklaven überführt, der im Verlaufe von 90 Minuten auf 175 °C erhitzt wird. Das Reaktionsgemisch wird 120 h bei dieser Temperatur und unter autogenem Druck und Rühren belassen. Nach Abkühlen auf Raumtemperatur und Öffnen des Autoklaven wird das kristallisierte Produkt auf einer Fritte von der Mutterlauge getrennt, mehrfach mit destilliertem Wasser gewaschen und nachfolgend bei 120 °C an der Luft 6 h getrocknet. Anschließend wird der Katalysator mit einer Aufheizgeschwindigkeit von 10 °C/min im Inertgasstrom (10 l/h) auf 550 °C geheizt und 4 h bei dieser Temperatur belassen und nachfolgend auf Raumtemperatur im Stickstoffstrom abgekühlt.
Das erhaltene Produkt wird als Katalysator A bezeichnet.

### Beispiel 3

Wie Beispiel 2, nur werden anstelle von 15 g Aktivkohle bei diesem Beispiel 20,7 g Aktivkohle zugegeben. Das hierbei erhaltene Produkt wird als Katalysator B bezeichnet.

### Beispiel 4

86,5 ml Wasserglas (347 g SiO₂/l) werden in 500 ml H₂O gelöst. Zu dieser Lösung werden 30 g A-Kohle (DARCO, Teilchengröße ≦ 32 µm) gegeben. Die erhaltene Suspension wird 30 min bei Raumtemperatur gerührt. Anschließend wird innerhalb von 30 min mit verdünnter H₂SO₄ (3,8 Masse-%) bis zum pH-Wert von 5,8 gefällt. Die erhaltene Fällsuspension wird 30 min bei Raumtemperatur nachgerührt. Das auf Aktivkohle gefällte SiO₂ wird filtriert, gewaschen und 24 h bei 80 °C an der Luft getrocknet.
Anschließend werden 8,84 g Tetraethylorthotitanat unter Inertgasbedingungen in 400 ml Ethanol gelöst. In diese klare Lösung wird das hergestellte Aktivkohle-SiO₂-Produkt eingetragen. Die dabei erhaltene Suspension wird in einem Vakuumrotationsverdampfer unter einem Vakuum von 16 mbar bis zur Trockene des Trägers behandelt. Der getränkte Träger wird in 690 g 20 %-iger Tetrapropylammoniumhydroxidlösung und 640 g Wasser suspendiert und in einen Autoklaven überführt, im Verlaufe von 90 min auf 175 °C erhitzt und 120 h bei dieser Temperatur belassen. Nach Abkühlen auf Raumtemperatur und Öffnen des Autoklaven wird das Produkt auf einem Filter von der Mutterlauge getrennt und mehrfach mit Wasser gewaschen. Nachfolgend wird das Produkt über einen Zeitraum von 6 h bei 120 °C an der Luft getrocknet und anschließend mit einer Aufheizgeschwindigkeit von 10 °C/min im Stickstoffstrom auf 550 °C geheizt, 4 h bei dieser Temperatur belassen und danach unter Stickstoffspülung auf Raumtemperatur abgekühlt. Der erhaltene Katalysator wird in der Tabelle 2 unter der Bezeichnung Katalysator C geführt.

### Beispiel 5

2,21 g Tetraethylorthotitanat wurden unter Rühren und Inertgasspülung zu 78,66 g Tetraethylorthosilikat gegeben. Nachfolgend setzt man diesem Gemisch, ebenfalls unter Inertgasspülung, 172,5 g 20 Masse-%-ige Tetrapropylammoniumhydroxidlösung zu. Dieses Gemisch wird unter Rühren eine Stunde bei 78 °C behandelt und mit 157,32 g Wasser verdünnt. In diese Lösung werden 15 g SiO₂ (Oberfläche: 385 m²/g, Teilchengröße ≦ 32 µm) eingetragen. Die homogene Suspension wird bei Raumtemperatur in einen teflonausgekleideten Autoklaven überführt und innerhalb von 90 min auf 175 °C erhitzt. Bei dieser Temperatur wird das Reaktionsgemisch unter autogenem Druck belassen. Nach Abkühlen auf Raumtemperatur und Öffnen des Autoklaven wird das Reaktionsprodukt auf einem Filter von der Mutterlauge getrennt, mehrfach mit destilliertem Wasser gewaschen und nachfolgend bei 120 °C an der Luft getrocknet. Nachfolgend wird das getrocknete Produkt mit einer Aufheizgeschwindigkeit von 10 °/min im Luftstrom (10 l/h) auf 550 °C geheizt und 2 h bei dieser Temperatur getempert. Anschließend wird der Katalysator auf Raumtemperatur abgekühlt.
Das erhaltene Produkt wird als Katalysator D bezeichnet.

### Beispiel 6

Wie Beispiel 5, anstelle von SiO₂ werden 15 g Al₂O₃.SiO₂ (20 Masse-% SiO₂, 226 m²/g, Teilchengröße ≦ 32 µm) als Träger verwendet.

Der Katalysator wird in der Tabelle 1 und 2 als Katalysator E bezeichnet.

### Beispiel 7

Wie Beispiel 5, anstelle von SiO₂ werden 17 g ZrO₂ (15,6 m²/g, Teilchengröße ≦ 32 µm) als Träger eingesetzt. Der Katalysator wird in der Tabelle 1 und 2 als Katalysator F angeführt.

### Beispiel 8

21,6 ml Wasserglas mit einem SiO₂-Gehalt von 347 g/l werden in 125 ml Wasser gelöst. Zu dieser Lösung werden 7,5 g γ-Al₂O₃ (186 m²/g, Teilchengröße ≦ 32 µm) gegeben. Diese Suspension wird 20 min bei Raumtemperatur gerührt und danach im Zeitraum von 20 min mit verdünnter H₂SO₄ (3,8 Masse-%) bis zum pH-Wert von 5,9 gefällt. Anschliessend wird die Suspension noch 30 min bei Raumtemperatur nachgerührt. Das auf Al₂O₃ gefällte SiO₂ wird filtriert, gewaschen und 6 h bei 120 °C an der Luft getrocknet. Danach werden 2,21 g Tetraethylorthotitanat schnell unter Inertgasspülung in 100 ml Ethylalkohol gelöst. In diese Lösung wird das hergestellte Al₂O₃-SiO₂-Produkt eingetragen. Die hierbei erhaltene Suspension wird in einem Vakuumreaktionsverdampfer unter einem Vakuum von ca. 16 mbar bis zur Trockene des Festkörper-Gemisches behandelt. Das Gemisch wird in 172,5 g 20 Masse-%-iger Tetrapropylammoniumhydroxid-Lösung und 160 g Wasser suspendiert und in einen teflonausgekleideten Autoklaven überführt. Diese Suspension wird innerhalb von 90 min auf 178 °C erhitzt und 120 h bei dieser Temperatur belassen. Nach Abkühlen auf Raumtemperatur und Öffnen des Autoklaven wird das Produkt durch Filtrieren von der Mutterlauge getrennt und mehrfach mit Wasser gewaschen. Anschließend wird das Produkt über einen Zeitraum von 6 h bei 120 °C an der Luft getrocknet, nachfolgend mit einer Aufheizrate von 10 °C/min an der Luft auf 550 °C geheizt, 3 h bei dieser Temperatur belassen und danach auf Raumtemperatur abgekühlt. Das erhaltene Produkt wird als Katalysator G bezeichnet.

### Beispiel 9

Wie Beispiel 8, anstelle des Al₂O₃ werden 16 g TiO₂ (21,0 m²/g, Teilchengröße ≦ 32 µm) eingesetzt. Das gebildete Produkt ist der Katalysator H.

### Beispiel 10

Wie Beispiel 8, anstelle des Al₂O₃ wird ein Gemenge aus 5 g ZrO₂, 4 g γ-Al₂O₃ und 6 g SiO₂ für die Katalysatorherstellung eingesetzt.
Das erhaltene Produkt wird in der Tabelle 2 als Katalysator J angegeben.

### Beispiel 11

Die Bestimmung der katalytischen Aktivität der erfindungsgemäßen Katalysatoren sowie des Vergleichskatalysators erfolgt durch deren Einsatz in der Umwandlung von Cyclohexanon mit Ammoniak und H₂O₂ zum Cyclohexanonoxim.

Dazu werden in ein Reaktionsgefäß jeweils 1,0 g Katalysator, 48 ml NH₃.H₂O (13,8 Masse-%) und 42 ml t-Butanol gegeben. Diese Suspension wird unter intensivem Rühren auf 80 °C hochgeheizt. Nach Erreichen der Reaktionstemperatur werden mittels zweier Dosiervorrichtungen 19 g H₂O₂ (30 %) und 17 g Cyclohexanon über einen Zeitraum von 270 min unter Rühren zur Vorlage gegeben. Anschliessend wird das Reaktionsgemisch noch 30 min bei Reaktionstemperatur gehalten und danach auf Raumtemperatur abgekühlt. Während der Reaktion stellt sich zu Beginn ein Überdruck von 84 bis 101 kPa (630 bis 760 Torr) ein, der am Ende der Reaktion auf einen Wert von ca. 40 kPa (ca. 300 Torr) zurückgeht.

Zur Aufarbeitung des Reaktionsgemisches wird der Katalysator abzentrifugiert und das flüssige Produkt mit 20 ml Cyclohexan, das vorher zum Spülen der Apparatur verwendet wurde und 20 g Ammoniumsulfat versetzt. Nach einer fünfminütigen Extraktion werden die Phasen getrennt, und die wäßrige Phase wird noch fünfmal mit je 10 ml Cyclohexan extrahiert. Die organischen Extrakte werden vereinigt und gaschromatographisch analysiert. Die Ergebnisse der Ausprüfung sind in der Tabelle 2 enthalten.

### Beispiel 12

In einen mit Stickstoff gespülten Autoklaven (200 ml Fassungsvermögen) werden 7,4 g n-Hexan, 17,8 g Aceton, 17,5 g H₂O₂ (35%ig) mit 0,25 g des Katalysators V bzw. 0,3 g des Katalysators H (entspricht einem Zeolithgehalt von 0,15 g) gegeben. Das Reaktionsgemisch wird 60 min bei 95 °C unter Rühren (600 Umdrehungen/min) umgesetzt und anschließend, nach Abkühlung auf Raumtemperatur, analysiert. Das n-Hexan wird dabei zu 1-Hexanol, 2-Hexanol, 2-Hexanon und 3-Hexanon umgewandelt. Der n-Hexanumsatz beträgt beim Katalysator V 54 % und beim erfindungsgemäßen Katalysator H 63 %.

### Beispiel 13

48 g 1-Heptanol, 80 g Aceton und 6,5 g des Katalysators V bzw. 12 g des erfindungsgemäßen Katalysators E (entspricht einem Zeolithgehalt von 5,8 g) werden in ein mit Rückflußkühler ausgerüstetes Reaktionsgefäß gegeben, auf 65 °C erwärmt, anschließend innerhalb von 30 min mit 10 g H₂O₂ (35%ig) versetzt und danach 5 h unter Rühren behandelt. Nach Abkühlung auf Raumtemperatur wird das Reaktionsgemisch analysiert. Am Katalysator V wurden 14,5 % und am erfindungsgemäßen Katalysator E 16,8 % des Heptanols zu Heptanal umgesetzt.

### Beispiel 14

12 g Allylalkohol, 30 g tert.-Butylalkohol, 100 g Wasser, 22 g H₂O₂ (35 %) werden mit 3,5 g des Katalysators V bzw. 6,9 g des erfindungsgemäßen Katalysators E (entspricht 3,4 g Zeolithanteil) in ein Reaktionsgefäß überführt und 8 h bei 30 °C gerührt. Danach wurde das Reaktionsgemisch analysiert. Am Katalysator V wurde eine Glycerinausbeute von 78,2 %, bezogen auf den eingesetzten Allylalkohol und am erfindungsgemäßen Katalysator E von 83,7 % gemessen.

### Beispiel 15

47 g Phenol, 10 g Wasser, 25 g Aceton, 16,3 g H₂O₂ (35 %) und 4,5 g des Katalysators V bzw. 7 g des erfindungsgemäßen Katalysators G (entspricht 3,4 g Zeolithanteil) werden in ein mit Rückflußkühler versehenes Reaktionsgefäß überführt und 5 h bei 60 °C gerührt. Anschließend wird das Reaktionsgemisch abgekühlt und analysiert. Die Analyse ergab einen Phenolumsatz von 28,9 % für den Katalysator V und 30,9 % für den erfindungsgemäßen Katalysator G. Die Reaktionsproduktzusammensetzung betrug in beiden Fällen 48 % Hydrochinon und 52 % Catechol.

### Beispiel 16

10 g 1-Octen und 40 g Methanol werden jeweils einmal mit 4,5 g Katalysator V bzw. 6 g Katalysator F (entspricht einem Zeolithanteil von 2,9 g) bzw. 6,4 g Katalysator J (entspricht einem Zeolithanteil von 3,2 g) in ein Reaktionsgefäß überführt und auf 48 °C erwärmt. Danach werden jeweils 5 g H₂O₂ (35 %) tropfenweise unter Rühren zugegeben. Nach 90 min Reaktionszeit wird das Reaktionsgemisch abgekühlt und analysiert. Dabei wurden folgende Ergebnisse erzielt:

| Katalysator | 1-Octenumsatz | Selektivität der 1,2-Epoxidbildung |
|---|---|---|
| V | 50,1 % | 92,5 % |
| F | 54,4 % | 93,4 % |
| J | 55,9 % | 93,5 % |

Die Beispiele 12 bis 16 verdeutlichen, daß die auf den Zeolithanteil bezogene Oxidationsaktivität der geträgerten Titansilikalite größer ist als die der ungeträgerten Zeolithphase.

**Tabelle 1**

| Katalysator | EZV in nm³ | Lage der IR-Bande (Si-O-Ti) in cm⁻³ |
|---|---|---|
| V | 5,3794 | 967 |
| A | 5,3390 | 947 |
| B | 5,3379 | 946 |
| C | 5,3501 | 949 |
| A 1 ¹⁾ | 5,3744 | 966 |
| D | 5,3632 | n.b. |
| E | 5,3594 | n.b. |
| F | 5,3580 | n.b. |
| G | 5,3619 | n.b. |
| H | 5,3625 | n.b. |
| n.b. = nicht bestimmt | | |

| | | |
|---|---|---|
| ¹⁾ A 1 wurde durch 20-stündiges vorsichtiges Abbrennen des Kohlenstoffes bei Temperaturen von 500 bis 550 °C aus dem Katalysator A erhalten. | | |

**Tabelle 2**

| Katalysator | Oximausbeute in % | Selektivität* in % |
|---|---|---|
| V | 88,7 | 94,6 |
| A | 92,8 | 98,4 |
| B | 91,3 | 98,1 |
| C | 91,0 | 98,3 |
| D | 92,4 | 98,1 |
| E | 90,9 | 98,0 |
| F | 91,4 | 98,3 |
| G | 92,1 | 98,1 |
| H | 92,2 | 97,9 |
| J | 91,7 | 98,5 |

| | | |
|---|---|---|
| * bezogen auf den Cyclohexanonumsatz | | |

## Patentansprüche

1. Oxidationskatalysatoren, bestehend aus auf Aktivkohle- oder Metalloxidträger in situ kristallisiertem Titansilikalit mit MFI-Struktur, mit einem Titansilikalitgehalt von 1 bis 90 Masse-% und einem Si:Ti-Atomverhältnis von 10 bis 100.

2. Oxidationskatalysatoren nach Anspruch 1, mit einem Tltansilikalitgehalt von 10 bis 90 Masse-%.

3. Oxidationskatalysatoren nach Anspruch 2, bestehend aus auf Aktivkohleträger kristallisiertem Titansilikalit, mit einem Titansilikalitgehalt von 40 bis 60 Masse-%.

4. Oxidationskatalysatoren nach Anspruch 2, bestehend aus auf Metalloxidträger kristallisiertem Titansilikalit, mit einem Titansilikalitgehalt von 30 bis 50 Masse-%.

5. Oxidationskatalysatoren nach einem der Ansprüche 1, 2 oder 4, mit Al₂O₃, SiO₂, TiO₂, ZrO₂ oder Al₂O₃ . SiO₂ oder Gemische dieser Oxide als Trägermaterial.

6. Oxidationskatalysatoren nach den Ansprüchen 1 bis 5 mit einem Elementarzellenvolumen der geträgerten Titansilikalitphase kleiner als das eines entsprechenden trägerfreien Titansilikalits.

7. Verfahren zur Herstellung von Oxidationskatalysatoren gemäß einem der Ansprüche 1 bis 3, durch
- Auffällen eines TiO₂-SiO₂-Gemisches auf Aktivkohle oder
- Auffällen von SiO₂ auf Aktivkohle und Auftränken einer Titanverbindung
und Behandlung im Autoklaven in Gegenwart eines Templates unter autogenem Druck bei Temperaturen von 150 bis 200 °C über einen Zeitraum von 24 bis 240 Stunden.

8. Verfahren zur Herstellung von Oxidationskatalysatoren gemäß einem der Ansprüche 1, 2 oder 4 durch
- Auftränken eines titanhaltigen Kieselsols auf Metalloxid
- Auffällen eines TiO₂-SiO₂-Gemisches auf Metalloxid oder
- Auffällen von SiO₂ auf Metalloxid und Auftränken einer Titanverbindung und
Behandlung im Autoklaven in Gegenwart eines Templates bei autogenem Druck und einer Temperatur von 150 bis 200 °C über einen Zeitraum von 48 bis 240 Stunden sowie anschliessendes Waschen, Filtrieren und Tempern.

9. Verwendung eines den Ansprüchen 1 bis 3 und 6 entsprechenden Oxidationskatalysators zur Herstellung von Oximen durch katalytische Umwandlung der entsprechenden Carbonylverbindung mit Ammoniak und Wasserstoffperoxid bei H₂O₂ : Carbonylverbindung-Molverhältnissen von 0,8 bis 1,2, NH₃ : Carbonylverbindung-Molverhältnissen von 1,2 bis 2,5, Temperaturen von 20 bis 120 °C, einem Druck gleich oder höher als atmosphärischer Druck, in Wasser oder einem organischen Lösungsmittel und unter intensivem Rühren.

10. Verwendung eines den Ansprüchen 1, 2 und 4 bis 6 entsprechenden Katalysators zur Herstellung von Oximen durch katalytische Umwandlung der entsprechenden Carbonylverbindung mit Ammoniak und Wasserstoffperoxid bei H₂O₂ : Carbonylverbindung-Molverhältnissen von 0,8 bis 2,0, NH₃ : Carbonylverbindungen-Molverhältnissen von 1,2 bis 2,5, Temperaturen von 70 bis 120 °C, einem Druck gleich oder höher als atmosphärischer Druck, in Wasser oder einem organischen Lösungsmittel und unter intensivem Rühren.

11. Verwendung eines den Ansprüchen 1 bis 6 entsprechenden Katalysators zur Hydroxylierung von Aromaten.

12. Verwendung eines den Ansprüchen 1, 2 und 4 bis 6 entsprechenden Katalysators zur Oxidation von gesättigten Kohlenwasserstoffen.

13. Verwendung eines den Ansprüchen 1 bis 6 entsprechenden Katalysators zur Oxidation von Olefinen.

14. Verwendung eines den Ansprüchen 1 bis 6 entsprechenden Katalysators zur Oxidation von Allylalkohol.

15. Verwendung eines den Ansprüchen 1 bis 6 entsprechenden Katalysators zur Oxidation von Alkoholen zu Aldehyden.

## Claims

1. Oxidation catalysts consisting of titanium silicalite with MFI structure crystallized in situ and carried on activated charcoal or metal oxide, with a titanium silicalite content of 1 to 90% by mass and a Si:Ti atomic ratio of 10 to 100.

2. Oxidation catalysts as in claim 1 with a titanium silicalite content of 10 to 90% by mass.

3. Oxidation catalysts as in claim 2 with a titanium silicalite content of 10 to 90% by mass, consisting of titanium silicalite crystallized on activated charcoal and a titanium silicalite content of 40% to 60% by mass.

4. Oxidation catalysts as claimed in claim 2, consisting of titanium silicalite crystallized and carried on metal oxide, with a titanium silicalite content of 30 to 50% by mass.

5. Oxidation catalysts as claimed in any one of claims 1, 2 or 4, with Al₂O₃, SiO₂, TiO₂, ZrO₂ or Al₂O₃ . SiO₂ or mixtures of these oxides as carrier material.

6. Oxidation catalysts as claimed in any one of claims 1 to 5 with an elementary cell volume of the carried titanium silicalite phase significantly smaller than that of a corresponding titanium silicalite without carrier.

7. Method of preparing oxidation catalysts as claimed in any one of claims 1 to 3, by
- precipitation of a TiO₂-SiO₂ mixture on activated charcoal or
- precipitation of SiO₂ on activated charcoal and impregnation of a titanium compound
and treatment in an autoclave in the presence of a template under autogenous pressure at temperatures of 150 to 200°C over a period of 24 to 240 hours.

8. Method of preparing oxidation catalysts in accordance with any one of claims 1, 2 or 4 by
- impregnating a titanium-containing silica sol on metal oxide,
- precipitation of a TiO₂-SiO₂ mixture on metal oxide, or
- precipitation of SiO₂ on metal oxide and impregnation of a titanium compound,
and treatment in an autoclave in the presence of a template at autogenous pressure and a temperature from 150 to 200°C over a period of 48 to 240 hours, followed by washing, filtration and tempering in a known manner.

9. Use of an oxidation catalyst in accordance with claims 1 to 3, and 6 for the preparation of oximes by catalytic transformation of the corresponding carbonyl compound with ammonia and hydrogen peroxide with H₂O₂:carbonyl compound molar ratios from 0.8 to 1.2, NH₃ : carbonyl compound molar ratios of 1.2 to 2.5, temperatures from 20 to 120°C, a pressure equal to or higher than atmospheric pressure in water or an organic solvent and with vigorous stirring.

10. Use of a catalyst in accordance with claims 1, 2 and 4 to 6 for the preparation of oximes by catalytic transformation of the corresponding carbonyl compound with ammonia and hydrogen peroxide with H₂O₂:carbonyl compound molar ratios from 0.8 to 2.0, NH₃ : carbonyl compound molar ratios from 1.2 to 2.5, temperatures from 20 to 120°C, a pressure equal to or higher than atmospheric pressure, in water or an organic solvent and with vigorous stirring.

11. Use of a catalyst in accordance with claims 1 to 6 for the hydroxylation of aromatic compounds.

12. Use of a catalyst in accordance with claims 1, 2 and 4 to 6 for the oxidation of saturated hydrocarbons.

13. Use of a catalyst in accordance with claims 1 to 6 for the oxidation of olefins.

14. Use of a catalyst in accordance with claims 1 to 6 for the oxidation of allyl alcohol.

15. Use of a catalyst in accordance with claims 1 to 6 for the oxidation of alcohols to aldehydes.

## Revendications

1. Catalyseurs d'oxydation se composant de silicalite de titane à structure MFI cristallisée in situ sur un support d'oxyde métallique ou de charbon actif ayant une teneur en silicalite de titane allant de 1 à 90% de la masse et ayant un rapport atomique Si : Ti allant de 10 à 100.

2. Catalyseurs d'oxydation selon la revendication 1 ayant une teneur en silicalite de titane allant de 10 à 90% de la masse.

3. Catalyseurs d'oxydation selon la revendication 2 se composant de silicalite de titane cristallisée sur un support de charbon actif ayant une teneur en silicalite de titane allant de 40 à 60% de la masse.

4. Catalyseurs d'oxydation selon la revendication 2 se composant de silicalite de titane cristallisée sur un support d'oxyde métallique ayant une teneur en silicalite de titane allant de 30 à 50% de la masse.

5. Catalyseurs d'oxydation selon l'une des revendications 1, 2 ou 4, ayant comme base Al₂O₃, SiO₂, TiO₂, ZrO₂ ou Al₂O₃ . SiO₂ ou des mélanges de ces oxydes.

6. Catalyseurs d'oxydation selon les revendications 1 à 5 ayant un volume de cellules élémentaires de la phase de silicalite de titane mise en support inférieur à la silicalite de titane correspondante exempte de support.

7. Procédé destiné à fabriquer des catalyseurs d'oxydation conformément à l'une des revendications 1 à 3 par
- précipitation d'un mélange de TiO₂-SiO₂ avec du charbon actif ou
- précipitation de SiO₂ avec du charbon actif et imprégnation d'un composé de titane
et autoclavage en présence d'une template sous pression autogène à des températures allant de 150 à 200°C dans un laps de temps allant de 24 à 240 heures.

8. Procédé destiné à fabriquer des catalyseurs d'oxydation conformément à l'une des revendications 1, 2 ou 4 par
- imprégnation d'un sol de silice titanifère avec un oxyde métallique
- précipitation d'un mélange de TiO₂-SiO₂ avec un oxyde métallique ou
- précipitation de SiO₂ avec un oxyde métallique et imprégnation d'un composé de titane et
autoclavage en présence d'une template sous pression autogène et à une température allant de 150 à 200°C dans un laps de temps allant de 48 à 240 heures et pour finir lavage, filtrage et trempe.

9. Utilisation d'un catalyseur d'oxydation correspondant aux revendications 1 à 3 et 6 destiné à fabriquer des oximes par transformation catalytique du composé carbonyle correspondant avec de l'ammoniaque et du peroxyde d'hydrogène pour des rapports moléculaires H₂O₂ : composé carbonyle allant de 0,8 à 1,2, pour des rapports moléculaires NH₃ : composé carbonyle allant de 1,2 à 2,5, à des températures allant de 20 à 120°C, pour une pression égale ou supérieure à la pression atmosphérique, dans de l'eau ou dans un solvant organique et en agitant vigoureusement.

10. Utilisation d'un catalyseur correspondant aux revendications 1, 2 et 4 à 6 destiné à fabriquer des oximes par transformation catalytique du composé carbonyle correspondant avec de l'ammoniaque et du peroxyde d'hydrogène pour des rapports moléculaires H₂O₂ : composé carbonyle allant de 0,8 à 2,0, pour des rapports moléculaires NH₃ : composé carbonyle allant de 1,2 à 2,5, à des températures allant de 20 à 120°C, pour une pression égale ou supérieure à la pression atmosphérique, dans de l'eau ou dans un solvant organique et en agitant vigoureusement.

11. Utilisation d'un catalyseur correspondant aux revendications 1 à 6 destiné à l'hydroxylation de séries aromatiques.

12. Utilisation d'un catalyseur correspondant aux revendications 1, 2 et 4 à 6 destiné à l'oxydation d'hydrocarbures saturés.

13. Utilisation d'un catalyseur correspondant aux revendications 1 à 6 destiné à l'oxydation d'oléfines.

14. Utilisation d'un catalyseur correspondant aux revendications 1 à 6 destiné à l'oxydation d'alcool allylique.

15. Utilisation d'un catalyseur correspondant aux revendications 1 à 6 destiné à l'oxydation d'aldéhydes.
